# EUROPEAN PATENT SPECIFICATION

(11) **EP 3 884 947 B1**
(45) Date of publication and mention of the grant of the patent: **15.11.2023**
(21) Application number: 21152314.7
(22) Date of filing: 02.12.2011
(51) Int. Cl.: A61K 31/485, A61K 31/137, A61P 3/04, A61K 45/06

(54) **INCREASING DRUG BIOAVAILABILITY IN NALTREXONE THERAPY**
ERHÖHUNG DER BIOVERFÜGBARKEIT VON HEILMITTELN IN EINER NALTREXONTHERAPIE
AUGMENTATION DE LA BIODISPONIBILITÉ PHARMACOLOGIQUE D'UN TRAITEMENT À BASE DE NALTREXONE

(30) Priority: 03.12.2010 US 41939510 P
(43) Date of publication of application: 29.09.2021
(62) Divisional of application: 17156985.8
(73) Proprietor: Nalpropion Pharmaceuticals LLC, Brentwood, TN 37027 (US)
(72) Inventor: FLANAGAN,, Shawn, San Diego,, CA California 92128 (US); DUNAYEVICH,, Eduardo, Westlake Village,, CA California 91362 (US)
(74) Representative: Carpmaels & Ransford LLP

(56) References cited:
- US-A1- 2008 110 792
- US-B2- 7 375 111
- US-B2- 7 682 633
- GREENWAY F.L. ET AL.: "Comparison of Combined Bupropion and Naltrexone Therapy for Obesity with Monotherapy and Placebo", JOURNAL OF CLINICAL ENDOCRINOLOGY AND METABOLISM, vol. 94, no. 12, December 2009 (2009-12), pages 4898-4906, XP055100385, US ISSN: 0021-972X, DOI: 10.1210/jc.2009-1350
- HAUSENLOY D.J.: "Contrave(TM): Novel treatment for obesity", CLINICAL LIPIDOLOGY 2009 FUTURE MEDICINE LTD. GBR, vol. 4, no. 3, June 2009 (2009-06), pages 279-285, XP009176559, ISSN: 1746-0875
- JOHNSON F. ET AL.: "Food effects on the pharmacokinetics of morphine sulfate and naltrexone hydrochloride extended release capsules", ADVANCES IN THERAPY, vol. 27, no. 11, 14 October 2010 (2010-10-14), pages 846-858, XP055104393, ISSN: 0741-238X, DOI: 10.1007/s12325-010-0074-x
- MIDHA K.K. ET AL.: "Exposure measures applied to the bioequivalence of two sustained release formulations of bupropion.", INTERNATIONAL JOURNAL OF CLINICAL PHARMACOLOGY AND THERAPEUTICS MAY 2005, vol. 43, no. 5, May 2005 (2005-05), pages 244-254, XP009176514, ISSN: 0946-1965

## Description

### BACKGROUND OF THE INVENTION

### Field of the Invention

The present invention relates to pharmaceutical compositions for use in therapy, comprising sustained release formulation of naltrexone or a pharmaceutically acceptable salt thereof and a sustained release formulation of bupropion or a pharmaceutically salt thereof, wherein said composition is administered with food.

### Description of the Related Art

Drug therapies utilizing naltrexone, including in a combination therapy with bupropion, are being investigated for the treatment of a variety of medical conditions, including overweight and obesity, cardiovascular risk factors, insulin resistance, food cravings, visceral fat conditions, smoking, and major depression. Despite the potential use of naltrexone in daily or otherwise regular therapies, currently approved prescribing information for naltrexone-containing products does not refer to studies that examine the effects of food on pharmacokinetics. Further, currently approved prescribing information for WELLBUTRIN SR^{®} characterizes the effects of food on bupropion exposure, but reports only an 11% increase in maximal plasma concentration (Cₘₐₓ) and a 17% increase in area under the concentration time curve (AUC). As a result, there is no guidance or restriction in the prescribing information for naltrexone, alone or in combination with bupropion, with respect to food or food effects.

### SUMMARY OF THE INVENTION

Unexpected food effects have now been identified for drug therapies that utilize naltrexone. Described herein are clinical trials that reveal that the administration of naltrexone and bupropion with food unexpectedly increases the bioavailability of each of these drugs, indicating a positive food effect. For example, the administration of a weight loss treatment comprising naltrexone and bupropion with a high fat, high calorie diet to an overweight or obese individual improves the Cₘₐₓ and AUC of each of these drugs, thereby improving the efficacy of the weight loss treatment. Although one would not ordinarily recommend, administer, or take a high fat, high calorie diet in conjunction with treatments that typically entail dietary restrictions (*e*.*g*., treatments for overweight or obesity, cardiovascular risk factors, insulin resistance, food cravings, or visceral fat conditions), the pharmaceutical compositions for use in therapy involve the administration of naltrexone and bupropion as defined in the claims with a wide range of foods.

In view of the observations described herein, a need exists for methods that account for positive food effects associated with naltrexone and bupropion combined therapies, including methods of increasing drug bioavailability, providing enhanced therapies, and providing information to individuals regarding these effects. Disclosed herein are pharmaceutical compositions for use in therapy which capitalize on these unexpected findings.

The claimed invention provides a pharmaceutical composition for use in therapy, comprising sustained release formulation of naltrexone or a pharmaceutically acceptable salt thereof and a sustained release formulation of bupropion or a pharmaceutically acceptable salt thereof, wherein said composition is administered with food.

The pharmaceutical compositions of the present invention can be used in a method of providing a weight loss regimen to an individual, comprising: identifying an individual in need of treatment for overweight or obesity; counseling the individual to make lifestyle changes comprising improved diet and exercise; and providing the individual with a composition comprising naltrexone or a pharmaceutically acceptable salt thereof in a sustained release formulation, and bupropion or a pharmaceutically acceptable salt thereof in a sustained release formulation, with instructions to take the composition with food.

The pharmaceutical compositions of the present invention can be used in a method of maximizing the efficacy of a treatment for overweight or obesity, comprising: reading a product label that contains information regarding the bioavailability of a composition when taken with or without food, where the composition comprises naltrexone or a pharmaceutically acceptable salt thereof in a sustained release formulation, and bupropion or a pharmaceutically acceptable salt thereof in a sustained release formulation; determining whether taking the composition with or without food increases the bioavailability of the composition based on the product label information; and administering the composition with food based on a determination that administering the composition with food increases the bioavailability of the composition.

The pharmaceutical compositions of the present invention can be used in a method of maximizing the efficacy of a treatment for overweight or obesity for a patient is provided, comprising: reading a product label that contains information regarding the bioavailability of a composition when taken with or without food, where the composition comprises naltrexone or a pharmaceutically acceptable salt thereof in a sustained release formulation and bupropion or a pharmaceutically acceptable salt thereof in a sustained release formulation; determining whether administering the composition with or without food increases the bioavailability of the composition based on the product label information; and instructing a patient to take the composition with food based on a determination that administering the composition with food increases the bioavailability of the composition.

The pharmaceutical compositions of the present invention can be used in a method of improving patient compliance with instructions to take a weight loss composition with food, comprising: providing a patient with a weight loss composition comprising naltrexone or a pharmaceutically acceptable salt thereof in a sustained release formulation, and bupropion or a pharmaceutically acceptable salt thereof in a sustained release formulation; instructing the patient to take the weight loss composition with food; and increasing the patient's compliance in taking the composition with food by informing the patient that taking the composition with food increases the bioavailability of the composition compared to taking the same amount of the composition without food.

The pharmaceutical compositions of the present invention can be used in a method of providing an FDA-approved weight loss drug, comprising supplying an individual with a composition comprising naltrexone or a pharmaceutically acceptable salt thereof in a sustained release formulation, and bupropion or a pharmaceutically acceptable salt thereof in a sustained release formulation, and a FDA-approved product label for the composition comprising information regarding the bioavailability of the composition when taken with or without food.

In any embodiment disclosed herein, the amount of the naltrexone or pharmaceutically acceptable salt thereof is in the range of about 4 mg to about 32 mg per day; the amount of the bupropion or pharmaceutically acceptable salt thereof is in the range of about 90 mg to about 360 mg per day; the naltrexone or pharmaceutically acceptable salt thereof comprises a non-sequestered formulation of naltrexone or a pharmaceutically acceptable salt thereof; the naltrexone or pharmaceutically acceptable salt thereof is administered concurrently with the bupropion or pharmaceutically acceptable salt thereof; the naltrexone or pharmaceutically acceptable salt thereof is administered prior to or subsequent to the bupropion or pharmaceutically acceptable salt thereof; the naltrexone or pharmaceutically acceptable salt thereof and the bupropion or pharmaceutically acceptable salt thereof are in a single dosage form; the single dosage form is selected from the group consisting of a pill, a tablet, and a capsule; the naltrexone or pharmaceutically acceptable salt thereof and the bupropion or pharmaceutically acceptable salt thereof are administered or are suitable for administration one or more times per day; the naltrexone or pharmaceutically acceptable salt thereof and the bupropion or pharmaceutically acceptable salt thereof are administered or are suitable for administration two or more times per day; the weight loss activity of the naltrexone or pharmaceutically acceptable salt thereof and the bupropion or pharmaceutically acceptable salt thereof is enhanced compared to the administration of the same amount of either compound alone; and/or the naltrexone or pharmaceutically acceptable salt thereof and the bupropion or pharmaceutically acceptable salt thereof are together in a single dosage form, where the single dosage form comprises, or comprises about, 4 mg, 8 mg, or 16 mg of the naltrexone or pharmaceutically acceptable salt thereof and comprises, or comprises about, 90 mg or 180 mg of the bupropion or pharmaceutically acceptable salt thereof, and where each of the naltrexone or pharmaceutically acceptable salt thereof and the bupropion or pharmaceutically acceptable salt thereof is in a sustained release formulation.

The bioavailability of the naltrexone or pharmaceutically acceptable salt thereof is increased compared to the bioavailability of the same amount of the naltrexone or pharmaceutically acceptable salt thereof administered without food; increasing the bioavailability comprises increasing the maximal plasma concentration (Cₘₐₓ) or the extent of absorption (AUC) of the naltrexone or pharmaceutically acceptable salt thereof; the increase in bioavailability comprises an increase in Cₘₐₓ in the range of about 91% to about 271% and an increase in AUC in the range of about 70% to about 107% for the naltrexone or pharmaceutically acceptable salt thereof when taken with a meal compared to the same amount of the naltrexone or pharmaceutically acceptable salt thereof taken during a fasted condition; the increase in bioavailability comprises about a 3.7-fold increase in Cₘₐₓ and about a 2.1-fold increase in AUC for the naltrexone or pharmaceutically acceptable salt thereof when taken with a meal compared to the same amount of the naltrexone or pharmaceutically acceptable salt thereof taken during a fasted condition; and/or the increase in bioavailability comprises about a 1.9-fold increase in Cₘₐₓ and about a 1.7-fold increase in AUC for the naltrexone or pharmaceutically acceptable salt thereof when taken with a meal compared to the same amount of the naltrexone or pharmaceutically acceptable salt thereof taken during a fasted condition.

In any embodiment disclosed herein, the pharmaceutical compositions of the invention for use in the claimed therapy may be administered such that the naltrexone or pharmaceutically acceptable salt thereof and the bupropion or pharmaceutically acceptable salt thereof are administered in multiple time-spaced doses, where at least one of the time-spaced doses is administered with food; where each of the time-spaced doses is administered with food; the naltrexone or pharmaceutically acceptable salt thereof is provided or administered in an amount in the range of, or of about, 4 mg to 32 mg per day; and/or the bupropion or pharmaceutically acceptable salt thereof is provided or administered in an amount in the range of, or of about, 90 mg to 360 mg per day.

In any embodiment disclosed herein, the food comprises a high-fat meal; and/or the food comprises a meal selected from the group consisting of a moderate-calorie, moderate-fat meal of, or of about, 575 calories and fat accounting for, or for about, 23% of the total calorie content, a high-calorie, high-fat meal of, or of about, 1000 calories and fat accounting for, or for about, 50% of the total calorie content, and a meal that falls within a range defined by the moderate-calorie, moderate-fat meal and the high-calorie, high-fat meal.

In any embodiment disclosed herein, the individual is overweight or obese; and/or the individual suffers from obesity or overweight, and a therapeutically effective amount of the pharmaceutical compositions of the invention is provided or administered to treat the obesity or overweight.

In any embodiment disclosed herein, the naltrexone or a pharmaceutically acceptable salt thereof is administered to the individual in accordance with instructions described herein; the printed information indicates that the increase in bioavailability comprises an increase in the Cₘₐₓ or AUC of the naltrexone or pharmaceutically acceptable salt thereof; the printed information indicates an increase in Cₘₐₓ between, or between about, 91% to 271% and an increase in AUC between, or between about, 70% to 107% for the naltrexone or pharmaceutically acceptable salt thereof when taken with a meal compared to the same amount of the naltrexone or pharmaceutically acceptable salt thereof taken during a fasted condition; the printed information further states that taking the naltrexone or pharmaceutically acceptable salt thereof with food results in an increase in the bioavailability of the naltrexone or pharmaceutically acceptable salt thereof compared to taking the same amount of the naltrexone or pharmaceutically acceptable salt thereof without food; and/or the printed information comprises information required by a governmental agency for sale of the kit.

Disclosed herein but not specifically recited in the claims is a kit which further comprises at least one dosage form comprising bupropion or a pharmaceutically acceptable salt thereof; the kit provides dosages for multiple days, where the dosage of the naltrexone or pharmaceutically acceptable salt thereof is in the range of, or of about, 4 mg to 32 mg per day; the kit provides dosages for multiple days, where the dosage of the bupropion or pharmaceutically acceptable salt thereof is in the range of, or of about, 90 mg to 360 mg per day; and/or the kit further comprises a container, where the container comprises the at least one dosage form, and where the printed information is associated with the container.

In any embodiment disclosed herein, the treatment schedule for the naltrexone or pharmaceutically acceptable salt thereof and the bupropion or pharmaceutically acceptable salt thereof is: a first amount of the naltrexone or pharmaceutically acceptable salt thereof and the bupropion or pharmaceutically acceptable salt thereof for a first treatment period; a second amount of the naltrexone or pharmaceutically acceptable salt thereof and the bupropion or pharmaceutically acceptable salt thereof for a second treatment period, where the second amount comprises, or comprises about, twice as much of the naltrexone or pharmaceutically acceptable salt thereof and, or and about, twice as much of the bupropion or pharmaceutically acceptable salt thereof as the first amount; a third amount of the naltrexone or pharmaceutically acceptable salt thereof and the bupropion or pharmaceutically acceptable salt thereof for a third treatment period, where the third amount comprises, or comprises about, three times as much of the naltrexone or pharmaceutically acceptable salt thereof and, or and about, three times as much of the bupropion or pharmaceutically acceptable salt thereof as the first amount; and a fourth amount of the naltrexone or pharmaceutically acceptable salt thereof and the bupropion or pharmaceutically acceptable salt thereof for a fourth treatment period, where the fourth amount comprises, or comprises about, four times as much of the naltrexone or pharmaceutically acceptable salt thereof and, or and about, four times as much of the bupropion or pharmaceutically acceptable salt thereof as the first amount.

In any embodiment disclosed herein, the treatment schedule for the naltrexone or pharmaceutically acceptable salt thereof and the bupropion or pharmaceutically acceptable salt thereof is, or is about, 8 mg of the naltrexone or a pharmaceutically acceptable salt thereof and is, or is about, 90 mg of the bupropion or a pharmaceutically acceptable salt thereof for the first week of treatment; is, or is about, 16 mg of the naltrexone or a pharmaceutically acceptable salt thereof and is, or is about, 180 mg of the bupropion or a pharmaceutically acceptable salt thereof for the second week of treatment; is, or is about, 24 mg of the naltrexone or a pharmaceutically acceptable salt thereof and is, or is about, 270 mg of the bupropion or a pharmaceutically acceptable salt thereof for the third week of treatment; and is, or is about, 32 mg of the naltrexone or a pharmaceutically acceptable salt thereof and is, or is about, 360 mg of the bupropion or a pharmaceutically acceptable salt thereof for the fourth and any subsequent weeks of treatment.

In any embodiment disclosed herein, the naltrexone or pharmaceutically acceptable salt thereof and the bupropion or pharmaceutically acceptable salt thereof are administered as two 8 mg tablets of sustained-release naltrexone twice daily and two 90 mg tablets of sustained-release bupropion twice daily.

In any embodiment disclosed herein, the naltrexone or pharmaceutically acceptable salt thereof and the bupropion or pharmaceutically acceptable salt thereof are administered for at least 28 weeks; or the naltrexone or pharmaceutically acceptable salt thereof and the bupropion or pharmaceutically acceptable salt thereof are administered for at least 56 weeks.

The invention can be used in the preparation of a medicament for treating overweight or obesity as described in any of the embodiments disclosed herein.

The invention can be used in the preparation of a medicament for increasing drug bioavailability in combined naltrexone/bupropion weight loss therapy as described in any of the embodiments disclosed herein.

The invention can be used in the preparation of a medicament for providing enhanced naltrexone therapy to an individual as described in any of the embodiments disclosed herein.

The invention can be used in the preparation of a medicament for providing a weight loss regimen to an individual as described in any of the embodiments disclosed herein.

The invention can be used in the preparation of a medicament for maximizing the efficacy of a treatment for overweight or obesity as described in any of the embodiments disclosed herein.

The invention can be used in the preparation of a medicament for improving patient compliance with instructions to take a weight loss composition with food as described in any of the embodiments disclosed herein.

The invention can be used in the preparation of a medicament for providing an FDA-approved weight loss drug as described in any of the embodiments disclosed herein.

An embodiment of the invention includes a pharmaceutical composition for treating overweight or obesity as described in any of the embodiments disclosed herein comprising a sustained release formulation of naltrexone or a pharmaceutically acceptable salt thereof and a sustained release formulation of bupropion or a pharmaceutically acceptable salt thereof, wherein said composition is administered with food.

An embodiment of the invention includes a pharmaceutical composition for increasing drug bioavailability in combined naltrexone/bupropion weight loss therapy as described in any of the embodiments disclosed herein comprising a sustained release formulation of naltrexone or a pharmaceutically acceptable salt thereof and a sustained release formulation of bupropion or a pharmaceutically acceptable salt thereof, wherein said composition is administered with food.

An embodiment of the invention includes a pharmaceutical composition for providing enhanced naltrexone therapy to an individual as described in any of the embodiments disclosed herein comprising a sustained release formulation of naltrexone or a pharmaceutically acceptable salt thereof and a sustained release formulation of bupropion or a pharmaceutically acceptable salt thereof, wherein said composition is administered with food.

An embodiment of the invention includes a pharmaceutical composition for providing a weight loss regimen to an individual as described in any of the embodiments disclosed herein comprising a sustained release formulation of naltrexone or a pharmaceutically acceptable salt thereof and a sustained release formulation of bupropion or a pharmaceutically acceptable salt thereof, wherein said composition is administered with food.

An embodiment of the invention includes a pharmaceutical composition for maximizing the efficacy of a treatment for overweight or obesity as described in any of the embodiments disclosed herein comprising a sustained release formulation of naltrexone or a pharmaceutically acceptable salt thereof and a sustained release formulation of bupropion or a pharmaceutically acceptable salt thereof, wherein said composition is administered with food.

An embodiment of the invention includes a pharmaceutical composition for improving patient compliance with instructions to take a weight loss composition with food as described in any of the embodiments disclosed herein comprising a sustained release formulation of naltrexone or a pharmaceutically acceptable salt thereof and a sustained release formulation of bupropion or a pharmaceutically acceptable salt thereof, wherein said composition is administered with food.

An embodiment of the invention includes a pharmaceutical composition for providing an FDA-approved weight loss drug as described in any of the embodiments disclosed herein comprising a sustained release formulation of naltrexone or a pharmaceutically acceptable salt thereof and a sustained release formulation of bupropion or a pharmaceutically acceptable salt thereof, wherein said composition is administered with food.

### DETAILED DESCRIPTION OF THE PREFERRED EMBODIMENT

Various embodiments described herein increase the bioavailability of naltrexone and/or bupropion in naltrexone and combined naltrexone/bupropion therapies. Increasing the bioavailability of naltrexone and/or bupropion can improve a variety of therapies. For example, increased bioavailability can result in more effective dosing. In some embodiments, more effective dosing allows for a lower dosage of naltrexone and/or bupropion to be administered to an individual. In some embodiments, administration of naltrexone and/or bupropion with food can also reduce the frequency and/or severity of adverse effects associated with naltrexone, bupropion, or other drugs. In some embodiments, a reduction in side effects results in improved patient compliance with a treatment. The administration of naltrexone or combined naltrexone/bupropion therapies with food can also generally improve the consistency of pharmacokinetics associated with naltrexone or combined naltrexone/bupropion therapies as variability tends to be highest when bioavailability is low. In some embodiments, this improved consistency allows for greater dosing certainty and improved safety and/or tolerability for naltrexone, bupropion, or other drugs.

The term "bupropion" may be used in a general way herein to refer to a free base of bupropion, a pharmaceutically acceptable bupropion salt (including anhydrous forms, *e*.*g*., anhydrous bupropion), a bupropion metabolite (*e*.*g*., hydroxybupropion, threohydrobupropion, and erythrohydrobupropion), a bupropion isomer, or mixtures thereof. Reference herein to "bupropion" will be understood as encompassing all such forms, unless the context clearly indicates otherwise.

The term "naltrexone" may be used in a general way herein to refer to a free base of naltrexone, a pharmaceutically acceptable naltrexone salt (including hydrates and anhydrous forms, *e*.*g*., naltrexone hydrochloride dihydrate and anhydrous naltrexone hydrochloride), a naltrexone metabolite, a naltrexone isomer, or mixtures thereof. Reference herein to "naltrexone" will be understood as encompassing all such forms, unless the context clearly indicates otherwise.

The term "pharmaceutically acceptable salt," as used herein, refers to a formulation of a compound that does not cause significant irritation to an organism to which it is administered and does not abrogate the biological activity and properties of the compound. Pharmaceutical salts can be obtained by routine experimentation. Non-limiting examples of pharmaceutically acceptable salts include bupropion hydrochloride, radafaxine hydrochloride, naltrexone hydrochloride, and 6-β naltrexol hydrochloride.

Throughout the present disclosure, when a particular compound is mentioned by name, for example, bupropion or naltrexone, it is understood that the scope of the present disclosure encompasses pharmaceutically acceptable salts, esters, amides, or metabolites of the named compound. For example, in any of the embodiments herein, an active metabolite of naltrexone (*e*.*g*., 6-β naltrexol) can be used in combination with, or instead of, naltrexone. In any of the embodiments herein, an active metabolite of bupropion, including S,S-hydroxybupropion (*i*.*e*., radafaxine), can be used in combination with, or instead of, bupropion.

The term "sustained release," as used herein, has its ordinary meaning as understood by those skilled in the art and thus includes, by way of non-limiting example, the controlled release of a drug from a dosage form over an extended period of time. For example, in some embodiments, sustained-release dosage forms are those that have a release rate that is slower that of a comparable immediate release form, *e*.*g*., less than 80% of the release rate of an immediate-release dosage form.

Those skilled in the art will understand that an immediate-release naltrexone formulation appropriate for use as a reference standard is the immediate-release naltrexone formulation, widely available commercially as the REVIA^{®} brand of naltrexone hydrochloride, or an equivalent thereof. Those skilled in the art will also understand that an immediate-release bupropion formulation appropriate for use as a reference standard is the immediate-release bupropion formulation, widely available commercially as the WELLBUTRIN^{®} brand of bupropion, or an equivalent thereof. The U.S. government regulates the manner in which prescription drugs can be labeled and thus reference herein to the REVIA^{®} brand of naltrexone hydrochloride and WELLBUTRIN^{®} brand of bupropion have well-known, fixed, and definite meanings to those skilled in the art.

The term "oral dosage form," as used herein, has its ordinary meaning as understood by those skilled in the art and thus includes, by way of non-limiting example, a formulation of a drug or drugs in a form administrable to a human, including pills, tablets, cores, capsules, caplets, loose powder, solutions, and suspensions.

The term "food effect," as used herein, refers to a phenomenon that can influence the absorption of drugs following administration. A food effect can be designated "negative" when absorption is decreased, or "positive" when absorption is increased and manifested as an increase in bioavailability (*e*.*g*., as reflected by AUC). Food effects can also refer to changes in maximum concentration (Cₘₐₓ), or the time to reach maximum concentration (Tₘₐₓ), independently of overall absorption. As a result, some drugs can preferably be taken in either fasted or fed conditions to achieve an optimum desired effect. As used herein, the terms "with food" and "fed" can be used interchangeably. As used herein, the terms "without food," "fasted," and "fasting" can be used interchangeably.

The terms "mitigate" or "mitigation" of weight gain, as used herein, include preventing or decreasing the amount of weight gain associated, *e*.*g*., with the administration of a drug or a change in life activity. In some embodiments, mitigation of weight gain is measured relative to the amount of weight gain typically experienced when only one or neither of naltrexone or bupropion is administered.

The term "promotion" of weight loss, as used herein, includes causing weight loss relative to a baseline weight for a least a portion of the period of treatment. This includes an individual that initially gains some weight, but during the course of treatment loses weight relative to a baseline prior to beginning treatment, as well as individuals that regain a portion or all of the weight that is lost by the end of the treatment period. In a preferred embodiment, at the end of the treatment period, the individual has lost weight relative to a baseline. In a preferred embodiment, mitigation of weight gain or promotion of weight loss in a patient administered naltrexone and bupropion is greater than when neither or only one of naltrexone or bupropion is administered, and more preferably an at least additive, or better than additive, or synergistic, effect of administering the two compounds is achieved.

The terms "pharmacokinetic profile" or "pharmacokinetics," as used herein, have their ordinary meaning as understood by those skilled in the art and thus include, by way of non-limiting example, a characteristic of the curve that results from plotting concentration (e.g. blood plasma or serum) of a drug over time, following administration of the drug to a subject. A pharmacokinetic profile thus includes a pharmacokinetic parameter or set of parameters that can be used to characterize the pharmacokinetics of a particular drug or dosage form when administered to a suitable population. Various pharmacokinetic parameters are known to those skilled in the art, including area under the concentration vs. time curve (AUC), area under the concentration time curve from time zero until last quantifiable sample time (AUC₀₋ₜ), area under the concentration time curve from time zero extrapolated to infinity (AUC_{0-∞}), area under the concentration time curve over the steady state dosing interval or from time zero to twelve hours (AUC₀₋₁₂) for twice-daily dosing, maximum concentration (e.g. blood plasma/serum) after administration (Cₘₐₓ), and time to reach maximum concentration (e.g. blood plasma/serum) after administration (Tₘₐₓ). AUCₗₐₛₜ indicates the area under the blood plasma concentration vs. time curve from the time of administration until the time of the last time point. Pharmacokinetic parameters may be measured in various ways known to those skilled in the art, *e*.*g*., for single dosage or steady-state. Differences in one or more of the pharmacokinetic profiles (*e*.*g*., Cₘₐₓ) may indicate pharmacokinetic distinctness between two formulations.

The term "sequestered," as used herein, refers to a drug that is not substantially released following the administration of a dosage form comprising the drug. For example, dosage forms comprising morphine sulfate and naltrexone have been formulated in a manner that greatly reduces *in vivo* release of naltrexone following the administration of the intact version of the dosage form, *e*.*g*., as described in U.S. Patent Publication No. 2009/0162450.

Those skilled in the art will understand that pharmacokinetic parameters may be determined by comparison to a reference standard using clinical trial methods known and accepted by those skilled in the art, *e*.*g*., as described in the examples set forth herein. Since the pharmacokinetics of a drug can vary from patient to patient, such clinical trials generally involve multiple patients and appropriate statistical analyses of the resulting data (*e*.*g*., ANOVA at 90% confidence). Comparisons of pharmacokinetic parameters can be on a dose-adjusted basis, as understood by those skilled in the art.

Pharmacokinetic profiles can be determined by analyzing a patient population that has received a treatment of naltrexone alone, or a combined treatment of naltrexone and one or more other drugs (such as bupropion), with food, and comparing them to a comparable patient population that has received the same treatment without food. In some embodiments, the pharmacokinetic properties are measured after a single-drug dosage, while in others, they are measured at steady-state. In some embodiments, pharmacokinetics can be determined by monitoring a plasma naltrexone and/or active naltrexone metabolite (*e*.*g*., 6β-naltrexol) concentration profile. In some embodiments, pharmacokinetics can be determined by monitoring a plasma bupropion and/or active bupropion metabolite (*e*.*g*., threohydrobupropion) concentration profile.

An increase in bioavailability can be determined using one or more measures known to one of skill in the art, such as an increase in AUC, Cₘₐₓ, or Tₘₐₓ, which can each independently be an increase that is, is about, is at least, is at least about, 5%, 10%, 20%, 30%, 40%, 50%, 75%, 100%, 125%, 150%, 175%, 200%, 250%, 300%, 350%, 400%, 450%, 500%, 600%, 700%, or more, or within a range defined by any two of these values (*e.g*., 5%-500%, 10%-400%, or 20%-300%), wherein the increase is as compared to a reference treatment (*e*.*g*., a fasted state or a different fed state).

In preferred embodiments, the bioavailability of naltrexone, bupropion, or metabolites thereof falls outside the standard 80% - 125% range for bioequivalence. For example, the 90% confidence interval ("CI") for the Cₘₐₓ or AUC for naltrexone may be higher than 125% of the reference treatment (*e*.*g*., a fasted state or a different fed state).

Food taken with a naltrexone monotherapy or a combined therapy is preferably solid food with sufficient bulk and fat content to not be rapidly absorbed after consumption. In a preferred embodiment, the food taken is a meal.

In some embodiments, food is taken in a single dosing. In some embodiments, food is taken in repeated dosings. In some embodiments, the food is taken in repeated dosings until a steady state is reached. For example, food can be taken at regular intervals three times per day during treatment until the Cₘₐₓ of naltrexone and/or bupropion reaches a steady state.

In some embodiments, food is taken prior to, concurrently with, or subsequent to the administration of naltrexone or a combined naltrexone/bupropion therapy. In some embodiments, the time between the consumption of food and the administration of a naltrexone or a combined naltrexone/bupropion therapy is, is about, is not more than, is not more than about 5, 10, 15, 30, 45, or 60 minutes, or a range defined by any two of the preceding values. In some embodiments, the time between the consumption of food and the administration of naltrexone or a combined naltrexone/bupropion therapy is consistent, while in other embodiments, the time between the consumption of food and the administration of naltrexone or a combined naltrexone/bupropion therapy varies. In embodiments in which naltrexone and bupropion are taken separately, the time between the consumption of food and the administration of naltrexone may differ from the time between the consumption of food and the administration of bupropion.

In some embodiments, the act of providing includes supplying, acquiring, or administering (including self-administering) a composition described herein. In some embodiments, "providing" food includes an individual obtaining and consuming food on their own. For example, in some embodiments, an individual selects and purchases a meal that they consume in accordance with the methods provided herein.

Pharmaceutical compositions as defined in the claims for use in methods of increasing drug bioavailability in a combined naltrexone/bupropion therapy are provided. Also provided are pharmaceutical compositions as defined in the claims for use in methods of treating an individual using naltrexone, where the individual is provided information, preferably printed, regarding bioavailability and/or instructions to take the naltrexone or combined naltrexone/bupropion therapy with food. In some embodiments, the pharmaceutical compositions of the invention may be useful in methods of enhancing naltrexone therapy.

The invention is used during the administration of a treatment. The treatment may be effective to promote weight loss or mitigate weight gain in an overweight or obese individual. Obesity has been defined in terms of body mass index (BMI). BMI is calculated as weight (kg)/[height (m)]². According to the guidelines of the U.S. Centers for Disease Control and Prevention (CDC) and the World Health Organization (WHO), for adults over 20 years old, BMI falls into one of the following categories: below 18.5 is considered underweight, 18.5-24.9 is considered normal, 25.0-29.9 is considered overweight, and 30.0 and above is considered obese (World Health Organization. Physical status: The use and interpretation of anthropometry. Geneva, Switzerland: World Health Organization 1995. WHO Technical Report Series).

In some embodiments, the individual has a body mass index (BMI) of at least 25 kg/m². In some embodiments, the individual has a BMI of at least 30 kg/m². In some embodiments, the individual has a BMI of at least 40 kg/m². In some embodiments, the individual has a BMI of less than 25 kg/m², or develops a BMI less than 25 kg/m² during the course of administration of naltrexone and bupropion. In these embodiments, it may be beneficial for health or cosmetic purposes to mitigate subsequent weight gain or to promote weight loss, thereby reducing the BMI even further. In some embodiments, the individual has been diagnosed by a physician as being overweight or obese. In some embodiments, the individual is identified, including self-identified, as overweight or obese, or is identified as having been diagnosed as overweight or obese.

In some embodiments, the promotion of weight loss is measured by a percent change from a baseline body weight. In some of these embodiments, the amount of weight loss is, is about, is at least, is at least about 0.5%, 1%, 1.5%, 2%, 2.5%, 3%, 4%, 5%, 6%, 7%, 8%, 9%, 10%, 12%, 15%, or more of initial body weight, or a range defined by any two of the preceding values. In some embodiments, the promotion of weight loss is measured as a reduction in weight gain relative to the amount of weight gain experienced when neither or only one of naltrexone and bupropion is administered, and the amount of reduction in weight gain is, is about, is at least, is at least about, 2%, 5%, 10%, 15%, 20%, 25%, 30%, 40%, 50%, 60%, 70%, 80%, 90%, 100%, 105%, 110%, 115%, 120%, or more, or a range defined by any two of the preceding values.

In some embodiments, the mitigation of weight gain is measured by a percent change from a baseline body weight. In some of these embodiments, the amount of weight gain is, is about, is not more than, is not more than about 0%, 0.5%, 1%, 1.5%, 2%, 2.5%, 3%, 4%, 5%, 6%, 7%, 8%, 9%, 10%, or more of initial body weight, or a range defined by any two of the preceding values.

In some embodiments, the dosage of naltrexone and/or bupropion is adjusted so that the patient loses weight at a rate of about 3% of baseline body weight every six months. However, the rate of weight loss for a patient may be adjusted by the treating physician based on the patient's particular needs.

In some embodiments, the mitigation of weight gain or promotion of weight loss occurs by increasing satiety in the individual. In some embodiments, the mitigation of weight gain or promotion of weight loss occurs by suppressing the appetite of the individual. In some embodiments, the treatment comprises instituting a regimen of diet and/or increased activity.

In some embodiments, the naltrexone and bupropion therapy is in an amount sufficient to affect weight loss, reduce a cardiovascular risk factor, increase insulin sensitivity, reduce food cravings, treat a visceral fat condition, mitigate weight gain or promote weight loss during smoking cessation, or provide weight loss therapy in patients with major depression. Non-limiting examples of such methods of treatment are disclosed in U.S. Patent Nos. 7,375,111 and 7,462,626; in U.S. Patent Publication Nos. 2007/0275970, 2007/0270450, 2007/0117827, 2007/0179168, 2008/0214592, 2007/0128298, and 2007/0129283; in U.S. Patent Application Nos. 12/751970, 61/167486, and 61/293844; and in WO 2009/158114. In some embodiments, the cardiovascular risk factor includes one or more of the following: total cholesterol level, LDL cholesterol level, HDL cholesterol level, triglyceride level, glucose level, and insulin level. In some embodiments, the cardiovascular risk factor includes one or more of the following: total cholesterol level, HDL cholesterol level, and triglyceride level.

In some embodiments, the increased efficacy of a weight loss treatment described herein comprises an improvement in an outcome measure. For example, in some embodiments, the increased efficacy increases the amount of weight loss. In some embodiments, the increase in efficacy decreases the frequency or severity of adverse events, including but not limited to nausea, constipation, vomiting, dizziness, dry mouth, headache, and insomnia. In some embodiments, the increased efficacy improves another secondary endpoint, including but not limited to waist circumference, high-sensitivity C-reactive protein (hs-CRP) levels, triglyceride levels, HDL cholesterol levels or the ratio of LDL/HDL cholesterol levels. As one of skill in the art will recognize, in some circumstances, it is desirable to decrease waist circumference, hs-CRP levels, triglyceride levels, and the ratio of LDL/HDL cholesterol levels, and to increase HDL cholesterol levels. In some embodiments, the improvement in the outcome measure is, is about, is at least, or is at least about 1, 2, 3, 4, 5, 7, 10, 12, 15, 20 30, 40, 50, 60, 70, 80, 90, or 100%, or within a range defined by any two of these values.

In some embodiments, the amount of weight loss when the treatment is taken with a meal is significantly more than the amount of weight loss when the treatment is taken without food. In some embodiments, the amount of weight loss when the treatment is taken with a high-fat meal is significantly more than the amount of weight loss when the treatment is taken without food. In some embodiments, the amount of weight loss when the treatment is taken with a high-fat meal during a steady state of a combined naltrexone/bupropion therapy is significantly more than the amount of weight loss when the treatment is taken under the same conditions without food.

The exact formulation, route of administration, and dosage for the pharmaceutical compositions of the invention can be chosen by the individual physician in view of the patient's condition. (*See e.g*., Fingl et al. 1975, in "The Pharmacological Basis of Therapeutics," Ch. 1 p. 1).

In some embodiments, naltrexone or naltrexone and bupropion are each administered once per day. In some embodiments, naltrexone and bupropion are each divided into equal doses and administered more than once per day. In some embodiments, naltrexone and bupropion are each divided into unequal doses and administered more than once per day. In some embodiments, naltrexone and bupropion are divided into a different number of doses and are administered a different number of times per day. In one such embodiment, the dose of one of naltrexone or bupropion is divided, while the dose of the other is not.

In some embodiments, one or both of naltrexone and bupropion is administered one, two, three, four, or more times per day. Either or both compounds can be administered less than once per day, for example once every 2, 3, 4, 5, 6, 7, 8, 9, 10, 11, 12, 13, or 14 days, or every 1 or 2 weeks, or a range defined by any two of the preceding values. In some embodiments, the number of administrations per day is constant (*e*.*g*., one time per day). In other embodiments, the number of administrations is variable. The number of administrations may change depending on effectiveness of the dosage form, observed side effects, external factors (*e.g*., a change in another medication), or the length of time that the dosage form has been administered.

In some embodiments, the daily dose of naltrexone can range from about 4 mg to about 50 mg, or about 4 mg to about 32 mg, or about 8 mg to about 32 mg, or about 8 mg to about 16 mg. In some embodiments, the daily dose is about 4 mg, about 8 mg, about 12 mg, about 16 mg, about 32 mg, or about 48 mg of naltrexone, or a range defined by any two of the preceding values. The selection of a particular dosage may be based on the weight of the patient. The selection of a particular dosage may be based on the identity, dosage, and/or dosing schedule of another co-administered compound. However, in some embodiments, it may be necessary to use dosages outside these ranges. In some embodiments, the daily dose is administered in a single oral dosage form. In some embodiments, the daily dose of naltrexone is the same, and in some embodiments, the daily dose is different.

In some embodiments, the daily dose of bupropion can range from about 30 mg to about 500 mg, or about 30 mg to about 360 mg, or about 90 mg to about 360 mg. In some embodiments, the daily dose is about 30 mg, about 90 mg, about 180 mg, about 360 mg, or about 450 mg of bupropion, or a range defined by any two of the preceding values. The selection of a particular dosage may be based on the weight of the patient. The selection of a particular dosage may be based on the identity, dosage and/or dosing schedule of another co-administered compound. However, in some embodiments, it may be necessary to use dosages outside these ranges. In some embodiments, the daily dose is administered in a single oral dosage form. In some embodiments, the daily dose of bupropion is the same, and in some embodiments, the daily dose is different.

The compositions described herein may be distributed, provided to a patient for self-administration, or administered to an individual. The combined naltrexone/bupropion therapies may include a third compound.

In some embodiments, naltrexone and/or bupropion are provided or administered as an oral dosage form. In some embodiments, the oral dosage form is in the form of a pill, tablet, core, capsule, caplet, loose powder, solution, or suspension. In a preferred embodiment, the oral dosage form is in the form of a pill, tablet, or capsule. In some embodiments, the combined naltrexone/bupropion therapy is provided in a single oral dosage form. In some embodiments, the oral dosage form is in the form of a trilayer tablet as described in U.S. Patent Publication No. 2008/0113026.

In some embodiments, at least one of naltrexone and bupropion is administered with varying frequency during treatment. In some of these embodiments, the varying frequency comprises a decreased frequency over time. For example, one or both of naltrexone and bupropion can be initially administered more than once per day, followed by administration only once per day at a later point in treatment. In some embodiments, the daily dosage of at least one of naltrexone and bupropion is consistent despite the varying frequency of administration. For example, in some embodiments, two tablets of each of naltrexone and bupropion are initially administered twice per day, while four tablets of each of naltrexone and bupropion are administered once per day at a later point in treatment. Alternatively, in some embodiments, one or two tablets of each of naltrexone and bupropion are administered at a later point in treatment, where the one or two tablets have an equivalent total daily dosage as the two tablets each of naltrexone and bupropion initially administered twice per day.

In some embodiments, the naltrexone in the combination treatment, is not a sequestered form of naltrexone. In preferred embodiments, at least 50% of the naltrexone is released within 24 hours of administration.

In some embodiments, at least one of naltrexone or bupropion is administered in consistent daily dosages throughout the period of treatment. In some embodiments, at least one of naltrexone or bupropion is administered in varying daily dosages during the period of treatment. In some of these embodiments, the daily dosages comprise increasing daily dosages over time. In some of these embodiments, the daily dosages comprise decreasing daily dosages over time.

In some embodiments, naltrexone and bupropion are administered individually. In some embodiments, naltrexone and bupropion are administered in a single pharmaceutical composition comprising naltrexone and bupropion. In the invention, both naltrexone and bupropion are provided in sustained release formulations. For example, sustained release forms of naltrexone are described in U.S. Patent Publication No. 2007/0281021. In some embodiments, at least one of naltrexone or bupropion is administered with a physiologically acceptable carrier, diluent, or excipient, or a combination thereof. Non-limiting examples of naltrexone/bupropion combinations, formulations thereof, and methods of administering them are disclosed in U.S. Patent Nos. 7,375,111 and 7,462,626. Reference herein to the use or administration of naltrexone and naltrexone/bupropion combinations will be understood to include all modes of administration disclosed or referred to herein, including without limitation separate administration, administration in a single dosage form, administration in the form of salts, and/or metabolites, and/or administration in sustained release forms. Techniques for formulation and administration of the compounds of the instant application may be found in "Remington's Pharmaceutical Sciences," Mack Publishing Co., Easton, PA, 18th edition, 1990.

In some embodiments, naltrexone is administered prior to bupropion. In some embodiments, naltrexone is administered subsequent to bupropion. In some embodiments, naltrexone and bupropion are co-administered. As used herein, coadministration includes administration in a single dosage form, or separate dosage forms that are administered at, or nearly at, the same time.

In some embodiments, the administration of naltrexone and bupropion is continued for a period of, or of about, 1, 2, 3, 4, 6, 8, 10, 12, 16, 20, 24, 36, 48, or 52 weeks, or a range defined by any two of the preceding values. In some embodiments, the administration of naltrexone and bupropion is continued until the reduction in symptoms of a disease, disorder, or condition is stabilized for a period of, or of about, 1, 2, 3, 4, 5, 6, or more weeks, or a range defined by any two of the preceding values. For example, in some embodiments, the administration of a combined naltrexone/bupropion therapy is continued until the mitigation of weight gain or promotion of weight loss in an individual is stabilized for a period of, or of about, 1, 2, 3, 4, 5, 6, or more weeks, or a range defined by any two of the preceding values. In some embodiments, administration of naltrexone and bupropion is continued until the individual no longer needs a treatment.

In some embodiments, "administering" a drug includes an individual obtaining and taking a drug on their own. For example, in some embodiments, an individual obtains a drug from a pharmacy and self-administers the drug in accordance with the methods provided herein.

Disclosed herein but not specifically recited in the claims is aa kit which may include one or more unit dosage forms comprising naltrexone, bupropion, or naltrexone and bupropion. The unit dosage forms may be of an oral formulation. For example, the unit dosage forms may comprise pills, tablets, or capsules. The kit may include a plurality of unit dosage forms. The unit dosage forms may be in a container. The dosage forms may be single oral dosage forms comprising naltrexone and bupropion or pharmaceutically acceptable salts thereof.

The invention disclosed herein may include information. The information may be in a form prescribed by a governmental agency regulating the manufacture, use, or sale of pharmaceuticals, which notice is reflective of approval by the agency of the form of the drug for human or veterinary administration. Such information, for example, may be the labeling approved by the U.S. Food and Drug Administration for prescription drugs, or the approved product insert. The information can include required information regarding dose and dosage forms, administration schedules and routes of administration, adverse events, contraindications, warning and precautions, drug interactions, and use in specific populations (*see*, *e.g., 21 C.F.R. § 201.57*), and may be required to be present on or associated with the drug for sale of the drug. Dosage forms comprising a sustained-release naltrexone formulation of the invention formulated in a compatible pharmaceutical carrier may also be prepared, placed in an appropriate container, and labeled for treatment of an indicated condition. A kit as disclosed herein is for sale of a prescription drug requiring the approval of and subject to the regulations of a governmental agency, such as the Food and Drug Administration of the United States. The kit may comprise the label or product insert required by the agency, such as the FDA, for sale of the kit to consumers, for example in the U.S.

The information may comprise instructions to administer the unit dosage form at a dosage of about 4 mg, about 8 mg, about 12 mg, about 16 mg, about 32 mg, or about 48 mg of naltrexone or a pharmaceutically acceptable salt thereof. The information may comprise instructions to administer the unit dosage form at a dosage of about 30 mg, about 90 mg, about 180 mg, about 360 mg, or about 450 mg of bupropion or a pharmaceutically acceptable salt thereof. These instructions may be provided in a variety of ways. The information may comprise instructions about when to administer the unit dosage forms. For example, the information may comprise instructions about when to administer the unit dosage forms relative to the administration of another medication or food. In preferred embodiments, the information instructs an individual to take naltrexone, or naltrexone and bupropion, with food, preferably a meal.

The information, preferably printed, may comprise information that that taking naltrexone or a pharmaceutically acceptable salt thereof with food results in an increase in the bioavailability of naltrexone or a pharmaceutically acceptable salt thereof compared to taking the same amount of naltrexone or a pharmaceutically acceptable salt thereof without food. Information, preferably printed, that taking bupropion or a pharmaceutically acceptable salt thereof with food results in an increase in the bioavailability of bupropion or a pharmaceutically acceptable salt thereof compared to taking the same amount of bupropion or a pharmaceutically acceptable salt thereof without food, may also be provided. Information, preferably printed, that taking naltrexone and bupropion, or a pharmaceutically acceptable salts thereof, with food results in an increase in the bioavailability of naltrexone and/or bupropion, or a pharmaceutically acceptable salts thereof, compared to taking the same amount of naltrexone and bupropion, or a pharmaceutically acceptable salts thereof, without food, may also be provided. Information, preferably printed, that taking naltrexone, and/or bupropion or pharmaceutically acceptable salts thereof with food results in fewer or less severe drug associated adverse events than taking the same amount of naltrexone and bupropion, or a pharmaceutically acceptable salts thereof, without food, may also be provided. The adverse events may be gastrointestinal events. Information regarding bioavailability, adverse events, or instructions on administration regimes may be provided to a subject, a dosage form comprising the medication described in the information is provided to the subject, and the dosage form is administered in accordance to the information. In some embodiments the subject is a patient in need of the medication. In some embodiments the medication is administered as a therapy for a disease as described herein.

Instructions and/or information may be present in a variety of forms, including printed information on a suitable medium or substrate (*e*.*g*., a piece or pieces of paper on which the information is printed), computer readable medium (*e*.*g*., diskette, CD, etc. on which the information has been recorded), or a website address that may be accessed via the internet. Printed information may, for example, be provided on a label associated with a drug product, on the container for a drug product, packaged with a drug product, or separately given to the patient apart from a drug product, or provided in manner that the patient can independently obtain the information (*e*.*g*., a website). Printed information may also be provided to a medical caregiver involved in treatment of the patient. In some embodiments, the information is provided to a person orally.

Disclosed herein but not specifically recited in the claims is a therapeutic package suitable for commercial sale, which may optionally comprise a container. The container can be in any conventional shape or form as known in the art which is made of a pharmaceutically acceptable material, for example a paper or cardboard box, a glass or plastic bottle or jar, a re-sealable bag (*e.g*., to hold a "refill" of tablets for placement into a different container), or a blister pack with individual dosages for pressing out of the pack according to a therapeutic schedule. The container employed can depend on the exact dosage form involved, *e.g*., a conventional cardboard box would not generally be used to hold a liquid suspension. It is feasible that more than one container can be used together in a single package to market a single dosage form. For example, tablets may be contained in a bottle which is in turn contained within a box. Non-limiting examples of packs and dispensers as well as oral dosage forms are disclosed in U.S. Patent Publication Nos. 2008/0110792 and 2008/0113026.

The information can be associated with the container, for example, by being: written on a label (*e*.*g*., the prescription label or a separate label) adhesively affixed to a bottle containing a dosage form described herein; included inside a container as a written package insert, such as inside a box which contains unit dose packets; applied directly to the container such as being printed on the wall of a box; or attached as by being tied or taped, *e*.*g*., as an instructional card affixed to the neck of a bottle via a string, cord or other line, lanyard or tether type device. The information may be printed directly on a unit dose pack or blister pack or blister card.

### EXAMPLES

The examples below are non-limiting and are merely representative of various aspects of the invention.

### Example 1: Single Dose Naltrexone and Bupropion

A phase I, open label, randomized, single-dose, three-way crossover study was performed to assess the effects of food on the plasma pharmacokinetics of sustained release naltrexone ("naltrexone SR") / sustained release bupropion ("bupropion SR") combination trilayer tablets. Healthy adult volunteers (n=18; 15 males, 3 females; mean age = 37 y.o.; range = 21-59 y.o.) were randomized to receive each of three treatments under in a crossover design with a minimum 14-day washout between treatments. The treatments consisted of a single dose of either: (1) two naltrexone SR 8 mg / bupropion SR 90 mg tablets (*i*.*e*., two "NB 8/90 tablets") under fasted conditions; (2) two NB 8/90 tablets administered shortly after a standardized high-fat meal; or (3) two NB 8/90 tablets administered shortly after a standardized moderate-fat meal. Blood samples for determination of plasma concentrations for naltrexone, bupropion, and their respective metabolites were measured within 15 minutes predose (baseline), and at time points from 0.5-120 hours postdose.

A summary of food effect comparisons in subjects administered NB 8/90 tablets is also provided in Table 1. Table 2 presents the results of statistical comparisons between the fed (*i*.*e*., treatment 3) and fasted (*i*.*e*., treatment 1) conditions. Administration of the NB 8/90 tablets under high-fat conditions increased naltrexone Cₘₐₓ and AUC values by 271% and 107%, respectively (*i.e.,* 3.7-fold and 2. 1-fold the value of the fasted condition, respectively), and increased bupropion Cₘₐₓ and AUC by 80% and 38%, respectively (*i*.*e*., 1.8-fold and 1.4-fold the value of the fasted condition, respectively), than those observed with the same tablets administered under fasted conditions. The 90% CIs of the naltrexone and bupropion Cₘₐₓ and AUC percent geometric mean ratios did not fall within the 80% to 125% range. Median Tₘₐₓ values and apparent terminal elimination t_{1/2} for naltrexone were similar for naltrexone between fed and fasted conditions. For bupropion, median Tₘₐₓ was one hour shorter under fed conditions than fasted conditions; apparent terminal elimination t_{1/2} values were similar at 22.70 and 20.44 hr, respectively. This indicates that food does not decrease clearance, but rather increases absorption and/or decreases first pass effect.

With respect to metabolites, Cₘₐₓ was increased 52% for 6β-naltrexol, 26% for threohydrobupropion, and 40% for a pharmacologically weighted composite of bupropion metabolites (PAWC, a single total concentration for all bupropion related active metabolites adjusted for relative potency) under high-fat conditions compared to fasted conditions, and the upper bounds of the 90% CIs for the Cₘₐₓ percent geometric mean ratios of these metabolites were generally above 125%. AUC for all metabolites and PAWC, and Cₘₐₓ for hydroxybupropion and erythrohydrobupropion were comparable between the high-fat and fasted conditions, with all 90% CIs within 80% to 125% range.

Administration of the NB 8/90 tablets in conjunction with a high-fat standardized meal resulted in a positive food effect, with naltrexone Cₘₐₓ and AUC increasing approximately 300% and 100%, respectively, and bupropion Cₘₐₓ and AUC increasing approximately 80% and 40%, respectively. In addition, administration of the combination with food reduced the adverse events, especially gastrointestinal events such as nausea. Three of 18 patients in the fasted group experienced a drug-related adverse event (17%), while only one of 16 patients in the fed group experienced an adverse event (6%).

**Table 1. Summary of Food Effect Comparisons in Subjects Administered NB 8/90 Tablets (Percent Geometric Least-Squares Mean Ratios)**

| **Study** | **Parameter** | **Analyte** | **Comparison to Fasted State** | | **Comparison of High-Fat to Moderate-Fat Estimate (p-value)** |
|---|---|---|---|---|---|
| | | | **Moderate-Fat Estimate (p-value)** | **High-Fat Estimate (p-value)** | |
| Example 1: NB-233 | Cₘₐₓ (ng/mL) | Naltrexone | N/A | 370.57% (<.0001)* | N/A |
| | | Bupropion | | 179.74% (<.0001)* | |
| | AUC_{0-∞} (ng-hr/mL) | Naltrexone | | 207.01% (<.0001)* | |
| | | Bupropion | | 138.25% (<.0001)* | |
| Example 2: NB-236 | Cₘₐₓ (ng/mL) | Naltrexone | 180.53% (<.0001)* | 191.64% (0.0003)* | 105.17%# (0.5788) |
| | | Bupropion | 117.20% (0.0034)^{∗} | 127.97% (0.0002)^{∗} | 109.47%# (0.0724) |
| | AUC₀₋₁₂ (ng-hr/mL) | Naltrexone | 169.70% (<.0001)* | 169.97% (<.0001)* | 100.20%# (0.9688) |
| | | Bupropion | 109.70%# (0.0025)^{∗} | 111.94% (0.0009)^{∗} | 102.53%# (0.3627) |
| Example 3: NB-239 | Cₘₐₓ (ng/mL) | Naltrexone | 213.71% (0.0003)* | N/A | N/A |
| | | Bupropion | 98.75%# (0.8986) | | |
| | AUC_{0-∞} (ng-hr/mL) | Naltrexone | 180.23% (0.0043)* | | |
| | | Bupropion | 92.60% (0.4020) | | |
| Abbreviations: AUC = area under the concentration-time curve from time zero until last quantifiable sample time (0-t) or extrapolated to infinity (0-co), Cₘₐₓ = maximum plasma concentration; Moderate-Fat = moderate-calorie, moderate-fat prandial state; High-Fat = high-calorie, high-fat prandial state; N/A = not applicable; # = the 90% CI fell within the 80-125% bioequivalence range; * p≤0.05 | | | | | |

**Table 2. Statistical Comparisons of Plasma Naltrexone and Bupropion Pharmacokinetic Parameters under Fed and Fasted Conditions from Example 1 (n = 18)**

| **PK Parameter^{a}** | **Arithmetic Mean ± SD (%CV)^{b}** | | |
|---|---|---|---|
| | **NB 8/90 Fasted (Reference)** | **NB 8/90 Fed** | **Fed to Fasted %MR (90% CI)^{c}** |
| **Naltrexone** | | | |
| Cₘₐₓ (ng/mL) | 1.14 ± 0.704 (61.5%) | 4.00 ± 2.52 (63.0%) | 370.57 (315.66 - 435.02) |
| Tₘₐₓ (hr) | 2.00 (0.75, 6.00) | 2.00 (0.75, 6.00) | Not Applicable |
| t_{1/2} (hr) | 5.89 ± 2.50 (42.5%) | 4.71 ± 2.14 (45.5%) | Not Applicable |
| AUC₀₋ₜ (ng·hr/mL) | 7.98 ± 4.04 (50.6%) | 16.42 ± 8.70 (53.0%) | 211.50 (198.11 - 225.81) |
| AUC_{0-∞} (ng·hr/mL) | 8.39 ± 4.26 (50.7%) | 16.65 ± 8.70 (52.3%) | 207.01 (193.52 - 221.44) |

| **Bupropion** | | | |
|---|---|---|---|
| Cₘₐₓ (ng/mL) | 161 ± 52.1 (32.4%) | 293 ± 109 (37.2%) | 179.74 (159.99 - 201.93) |
| Tₘₐₓ (hr) | 3.00 (1.50, 4.02) | 2.00 (1.25, 4.00) | Not Applicable |
| t_{1/2} (hr) | 20.44 ± 7.53 (36.9%) | 22.70 ± 6.82 (30.0%) | Not Applicable |
| AUC₀₋ₜ (ng·hr/mL) | 1550.91 ± 549.08 (35.4%) | 2179.15 ± 705.46 (32.4%) | 139.50 (130.82 - 148.77) |
| AUC_{0-∞} (ng·hr/mL) | 1616.20 ± 574.35 (35.5%) | 2253.46 ± 718.18 (31.9%) | 138.25 (129.55 - 147.53) |
| a = Subject 7 Period 1 Treatment B was excluded due to vomiting. | | | |
| b = Tₘₐₓ is presented as Median (Minimum, Maximum) | | | |
| c = Calculated based on In-transformed parameters. | | | |
| Abbreviations: AUC = area under the concentration-time curve from time zero until last quantifiable sample time (0-t) or extrapolated to infinity (0-∞), Cₘₐₓ= maximum plasma concentration; n = Number of subjects; NB 8/90 Fasted = Two naltrexone SR 8 mg / bupropion SR 90 mg trilayer tablets administered under fasted conditions (Reference, Treatment A); NB 8/90 Fed= Two naltrexone SR 8 mg/ bupropion SR 90 mg trilayer tablets administered under fed conditions (Test 2, Treatment C); SD= Standard deviation; SR= Sustained release; t_{½}= t1/2, apparent terminal elimination half-life; Tₘₐₓ= time to reach maximum plasma concentration; 90% CI = 90 percent confidence interval; %CV = Percent coefficient of variation; %MR = Geometric least-squares mean ratio. | | | |

### Example 2: Food Effect on Steady-State Naltrexone and Bupropion

A phase I, open label, steady-state study was performed to assess the plasma pharmacokinetics of NB tablets under fed and fasted conditions. An extension of the study allowed for the evaluation of the effect of food on the pharmacokinetics of NB tablets. Dosing to steady state affords an opportunity to observe accumulation of bupropion and metabolites, and thus better estimate the magnitude of pharmacokinetic interactions expected after chronic administration.

Days 1-31 of the study were devoted to the primary investigation of metoprolol pharmacokinetics in the context of steady-state dosing with NB tablets. Subjects (n=18) received a metoprolol 50 mg IR tablet on Days 1 and 31. NB was dose escalated every week from a single NB 8/90 tablet per day on Day 3 to a maximum dose of NB 32/360 (two NB 8/90 tablets BID) starting on Day 24. In the clinic setting (Days 1-3 and 27-31), standardized meals were provided before dosing and pharmacokinetic sampling. These meals were moderate in fat and caloric content. Subjects were instructed to take study medication with food during the outpatient (Days 4-26) phase of the study.

After Day 31, subjects (n=11; 7 males, 4 females; mean age = 34 y.o.; range = 19-45 y.o.) were given the option to participate in a 3-day treatment extension period designed to assess the effects of food on naltrexone and bupropion pharmacokinetics. The evaluation was designed to enable comparisons between fed conditions (either a "moderate" meal, or a high-fat, high-calorie meal) and between both fed conditions and the fasted state. The treatment sequence of the extension study was as follows:

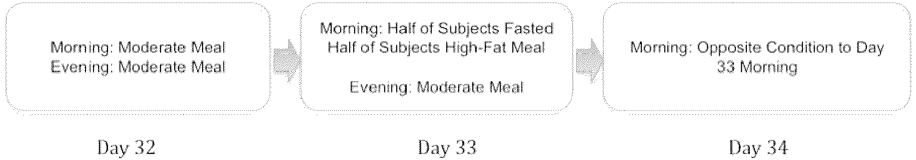

Naltrexone and bupropion pharmacokinetic data following a moderate meal was obtained on Day 30. Subjects enrolling in the optional extension continued BID dosing administration (with moderate meals) through Day 32. On Day 33, subjects were randomized in an approximate 1:1 ratio to receive the morning dose of study medication in either the fasted state or in the fed state with a high-fat (57%), high-calorie (910 kcal) meal. The evening dose on Day 33, study medication was given with food ("moderate" meal). For Day 34, subjects received their morning dose under the opposite condition from what they received on the morning of Day 33. On Days 33 and 34, pharmacokinetic samples were collected for naltrexone, bupropion, and their respective metabolites prior to dosing and through 12 hours of sampling following the morning dose. For purposes of studying the effects of food, the comparisons were between Day 30 pharmacokinetics ("moderate" meal) and the fasted and high-fat meal conditions administered on Day 33 and Day 34.

A summary of food effect comparisons in subjects administered NB 8/90 tablets is also provided in Table 1. Table 3 shows a summary and statistical comparison of naltrexone and bupropion steady state pharmacokinetic parameters under different meal conditions. Changing the meal composition prior to the morning dose from moderate-fat, moderate-calorie meal to high-fat, high-calorie meal had no effect on naltrexone Cₘₐₓ and AUC₀₋₁₂ parameter values, with the geometric mean ratios and CIs for the comparison contained within 80-125% bioequivalence range. However, the lower boundary of the 90% CI for the comparison of Cₘᵢₙ was below the 80% lower limit. The results for 6β-naltrexol were similar to those for naltrexone, with the exception that the 90% CIs for Cₘᵢₙ fell within the 80-125% range.

Administration of two NB 8/90 tablets following the moderate-fat, moderate-calorie meal and following the high-fat, high-calorie meal resulted in, respectively, 81% and 92%, higher naltrexone Cₘₐₓ values (*i.e*., 1. 8-fold and 1.9-fold the value of the fasted condition) and 70% higher (for both meals) AUC₀₋₁₂ values *(i.e.,* 1.7-fold the value of the fasted condition) relative to administration under fasting conditions. The effect on Cₘᵢₙ values was less and not consistent, with a 10% higher Cₘᵢₙ for the moderate-fat, moderate-calorie meal versus the fasted condition and a 12% lower Cₘᵢₙ for the high-fat, high-calorie meal versus the fasted condition. In contrast to the effects observed with naltrexone, neither meal type appeared to affect 6β-naltrexol exposure parameters. With the exception of Cₘₐₓ in the high-fat versus fasted conditions, which was slightly increased, all parameters and 90% CIs fell within the 80-125% range.

Changing the meal composition prior to the morning dose from a moderate-fat, moderate-calorie meal to a high-fat, high-calorie meal had no effect on bupropion absorption from two NB 8/90 tablets. The geometric mean ratios and 90% CIs for the comparisons of bupropion Cₘₐₓ, Cₘᵢₙ, and AUC₀₋₁₂ parameters following high-fat, high-calorie versus moderate-fat, moderate-calorie meal indicated a change of ~10% in Cₘₐₓ, and all parameters were entirely contained within the 80-125% bioequivalence range.

Administration of two NB 8/90 tablets following a moderate-fat, moderate-calorie meal and following a high-fat, high-calorie meal showed that food overall had no effect on the minimum and overall exposure to bupropion relative to administration in the fasted state, with the geometric mean ratios and 90% CIs for the comparisons of both Cₘᵢₙ and AUC₀₋₁₂ contained within the 80-125% bioequivalence range. Administration with food resulted in bupropion Cₘₐₓ falling just outside the bioequivalence range relative to administration in the fasted state, with the geometric mean ratios (90% CIs) of 117% (107.95% - 127.25%), respectively, for the moderate-fat, moderate-calorie condition versus the fasted condition, and 128% (118.72% - 137.95%), respectively, for the high-fat, high-calorie condition versus fasted condition. Prandial state had no effect on bupropion metabolites or on the PAWC, with all geometric mean ratios and 90% CIs falling within the 80-125% range.

In summary, in individuals at a steady state naltrexone/bupropion therapy, administration of two NB 8/90 tablets following a moderate-fat, moderate-calorie meal or following a high-fat, high-calorie meal resulted in substantially higher naltrexone Cₘₐₓ and AUC₀₋₁₂ values, and moderately higher bupropion Cₘₐₓ values, relative to the fasted state.

**Table 3. Summary and Statistical Analysis of Plasma Naltrexone and Bupropion Pharmacokinetic Parameters in the Extension Study from Example 2 (n = 11)**

| **PK Parameter** | **Arithmetic Mean ± SD (%CV)^{a}** | | |
|---|---|---|---|
| | **NB 8/90 Fed Moderate Meal Day 30** | **NB 8/90 Fasted Day 33 or 34** | **Fed to Fasted %MR (90% CI)^{b}** |
| **Naltrexone** | | | |
| Cₘₐₓ (ng/mL) | 2.60 ± 1.02 (39.4%) | 1.47 ± 0.710 (48.2%) | 180.53 (154.76 - 210.61) |
| Tₘₐₓ (hr) | 2.00 (1.00, 3.00) | 0.75 (0.75, 1.50) | Not Applicable |
| AUC₀₋ₜ (ng·hr/mL) | 11.74 ± 4.45 (37.9%) | 7.07 ± 2.88 (40.8%) | 169.70 (155.90 - 184.73) |
| Cₘᵢₙ (ng/mL) | 0.195 ± 0.105 (53.8%) | 0.172 ± 0.0758 (44.0%) | 110.04 (89.98 - 134.57) |

| **Bupropion** | | | |
|---|---|---|---|
| Cₘₐₓ (ng/mL) | 156 ± 52.5 (33.6%) | 131 ± 38.2 (29.1%) | 117.20 (107.95 - 127.25) |
| Tₘₐₓ (hr) | 3.00 (1.50, 4.00) | 1.99 (1.25, 3.00) | Not Applicable |
| AUC₀₋ₜ (ng·hr/mL) | 1055.81 ± 263.05 (24.9%) | 964.76 ± 249.16 (25.8%) | 109.70 (104.74 - 114.89) |
| Cₘᵢₙ (ng/mL) | 39.3 ± 9.61 (24.5%) | 41.6 ± 10.4 (24.9%) | 94.73 (89.52 - 100.25) |

| | **NB 8/90 Fed Moderate Meal Day 30** | **NB 8/90 Fed High-Fat Meal Day 33 or 34** | **High-fat to Moderate-fat %MR (90% CI)^{b}** |
|---|---|---|---|
| **Naltrexone** | | | |
| Cₘₐₓ (ng/mL) | 2.60 ± 1.02 (39.4%) | 2.84 ± 1.53 (53.9%) | 105.17 (90.15 - 122.69) |
| Tₘₐₓ (hr) | 2.00 (1.00, 3.00) | 3.00 (1.25, 6.00) | Not Applicable |
| AUC₀₋ₜ (ng·hr/mL) | 11.74 ± 4.45 (37.9%) | 11.79 ± 4.68 (39.7%) | 100.20 (92.04 - 109.07) |
| Cₘᵢₙ (ng/mL) | 0.195 ± 0.105 (53.8%) | 0.159 ± 0.0895 (56.4%) | 80.15 (65.54 - 98.02) |

| **Bupropion** | | | |
|---|---|---|---|
| Cₘₐₓ (ng/mL) | 156 ± 52.5 (33.6%) | 167 ± 43.4 (26.0%) | 109.47 (100.83 - 118.86) |
| Tₘₐₓ (hr) | 3.00 (1.50, 4.00) | 3.00 (1.50, 6.00) | Not Applicable |
| AUC₀₋ₜ (ng·hr/mL) | 1055.81 ± 263.05 (24.9%) | 1077.71 ± 244.24 (22.7%) | 102.53 (97.90 - 107.38) |
| Cₘᵢₙ (ng/mL) | 39.3 ± 9.61 (24.5%) | 38.8 ± 9.53 (24.6%) | 98.51 (93.09 - 104.24) |

| | **NB 8/90 Fasted Day 33 or 34** | **NB 8/90 Fed High-Fat Meal Day 33 or 34** | **Fed to fasted %MR (90% CI)^{b}** |
|---|---|---|---|
| **Naltrexone** | | | |
| Cₘₐₓ (ng/mL) | 1.47 ± 0.710 (48.2%) | 2.84 ± 1.53 (53.9%) | 191.64 (155.28 - 236.52) |
| Tₘₐₓ (hr) | 0.75 (0.75, 1.50) | 3.00 (1.25, 6.00) | Not Applicable |
| AUC₀₋ₜ (ng·hr/mL) | 7.07 ± 2.88 (40.8%) | 11.79 ± 4.68 (39.7%) | 169.97 (150.53 - 191.92) |
| Cₘᵢₙ (ng/mL) | 0.172 ± 0.0758 (44.0%) | 0.159 ± 0.0895 (56.4%) | 88.39 (69.26 - 112.80) |

| **Bupropion** | | | |
|---|---|---|---|
| Cₘₐₓ (ng/mL) | 131 ± 38.2 (29.1%) | 167 ± 43.4 (26.0%) | 127.97 (118.72 - 137.95) |
| Tₘₐₓ (hr) | 1.99 (1.25, 3.00) | 3.00 (1.50, 6.00) | Not Applicable |
| AUC₀₋ₜ (ng·hr/mL) | 964.76 ± 249.16 (25.8%) | 1077.71 ± 244.24 (22.7%) | 111.94 (107.31 - 116.77) |
| Cₘᵢₙ (ng/mL) | 41.6 ± 10.4 (24.9%) | 38.8 ± 9.53 (24.6%) | 92.79 (88.12 - 97.70) |
| a = Tₘₐₓ is presented as Median (Minimum, Maximum) | | | |
| b = Calculated based on In-transformed parameters. | | | |
| Abbreviations: AUC = area under the concentration-time curve from time zero until last quantifiable sample time (0-t) or extrapolated to infinity (0-∞), Cₘₐₓ = maximum plasma concentration; n = Number of subjects; NB 8/90 Fed (Moderate Meal) = Two naltrexone SR 8 mg/bupropion SR 90 mg trilayer tablets BID, administered under fed conditions (Treatment D24); NB 8/90 Fasted = Morning dose of two naltrexone SR 8 mg/bupropion SR 90 mg trilayer tablets administered under fasted conditions; NB 8/90 Fed (High-Fat Meal) = Morning dose of two naltrexone SR 8 mg/bupropion SR 90 mg trilayer tablets, administered under fed conditions; SD = Standard deviation; SR = Sustained release; t_{½} = t1/2, apparent terminal elimination half-life; Tₘₐₓ = time to reach maximum plasma concentration; 90% CI= 90 percent confidence interval; %CV = Percent coefficient of variation; %MR = Geometric least-squares mean ratio. | | | |

### Example 3: Naltrexone and Bupropion with a Moderate-Fat, Moderate-Calorie Meal

Two open label, crossover studies were compared to assess the effects of a moderate-fat, moderate-calorie meal to the fasted state on naltrexone and bupropion pharmacokinetics after a single dose. Healthy adult volunteers in a first open label single-dose crossover study (n=20) received two NB 8/90 tablets under a fasted condition. Healthy adult volunteers in a second open label, single-dose crossover study (n=18) received two NB 8/90 tablets administered shortly after a moderate-fat (23%), moderate-calorie (575 kcal) meal (analogous to dietary conditions in Phase 3 trials for weight loss with a combined naltrexone/bupropion therapy).

A summary of food effect comparisons in subjects administered NB 8/90 tablets is also provided in Table 1. Table 4 presents the results of statistical comparisons between the fed *(i.e.,* the second study) and fasted (*i.e*., the first study) conditions. The moderate-fat, moderate-calorie meal increased naltrexone Cₘₐₓ and AUC_{0-∞} by 114% and 80%, respectively, compared to the fasted condition (*i.e.,* 2.1-fold and 1.8-fold the value of the fasted condition, respectively). Bupropion pharmacokinetics were not significantly affected by this meal type, with somewhat lower Cₘₐₓ and AUC under fed conditions as compared to fasting conditions. The effect of the moderate-fat, moderate-calorie meal on naltrexone and bupropion pharmacokinetics was thus less marked than the food effect after a high-fat, high-calorie meal under single dose conditions, but remained statistically significant for naltrexone.

**Table 4. Exploratory Statistical Comparisons of Plasma Naltrexone and Bupropion Pharmacokinetic Parameters under Fed and Fasted Conditions from Example 3 (Evaluable Population, n = 18)**

| **PK Parameter ^{a}** | **Arithmetic Mean ± SD (%CV)^{b}** | | |
|---|---|---|---|
| | **NB 8/90 Fasted** | **NB 8/90 Fed** | **%MR (90% CI)^{c}** |
| **Naltrexone** | | | |
| Cₘₐₓ (ng/mL) | 1.34 ± 0.763 (57.0%) | 2.65 ± 1.18 (44.5%) | 213.71 (156.28 - 292.23) |
| Tₘₐₓ (hr) | 1.27 (0.75, 4.00) | 2.00 (0.75, 6.00) | Not Applicable |
| t_{1/2} (hr) | 5.07 ± 3.87 (76.4%) | 3.70 ± 1.85 (50.0%) | Not Applicable |
| AUC₀₋ₜ (ng·hr/mL) | 8.30 ± 6.41 (77.2%) | 13.41 ± 6.93 (51.6%) | 184.22 (132.93 - 255.30) |
| AUC_{0-∞} (ng·hr/mL) | 8.55 ± 6.46 (75.6%) | 13.63 ± 7.15 52.4%) | 180.23 (130.60 - 248.71) |

| **Bupropion** | | | |
|---|---|---|---|
| Cₘₐₓ (ng/mL) | 156 ± 36.9 (23.7%) | 154 ± 55.4 (35.9%) | 98.75 (83.59 - 116.65) |
| Tₘₐₓ (hr) | 2.07 (1.50, 4.01) | 3.02 (1.00, 6.00) | Not Applicable |
| t_{1/2} (hr) | 19.83 ± 3.24 (16.3%) | 15.87 ± 2.84 (17.9%) | Not Applicable |
| AUC₀₋ₜ (ng·hr/mL) | 1496.01 ± 323.04 (21.6%) | 1303.75 ± 310.06 (23.8%) | 91.55 (78.65 - 106.56) |
| AUC_{0-∞} (ng·hr/mL) | 1574.93 ± 346.49 (22.0%) | 1384.85 ± 330.19 (23.8%) | 92.60 (79.41 - 107.97) |
| a = Subject 1 in Study NB-239 was excluded due to vomiting. Subject 9 in Study NB-237 was excluded due to vomiting. | | | |
| b = Tₘₐₓ is presented as Median (Minimum, Maximum) | | | |
| c = Calculated based on In-transformed parameters. | | | |
| Abbreviations: AUC = area under the concentration-time curve from time zero until last quantifiable sample time (0-t) or extrapolated to infinity (0-∞), Cₘₐₓ = maximum plasma concentration; n = Number of subjects; NB 8/90 Fasted = Two naltrexone SR 8 mg/bupropion SR 90 mg trilayer tablets administered under fasted conditions (Reference Treatment D); NB 8/90 Fed = Two naltrexone SR 8 mg/bupropion SR 90 mg trilayer tablets administered with moderate-calorie, moderate-fat meal (Reference Treatment B); SD = Standard deviation; SR = Sustained release; t_{½} = t1/2, apparent terminal elimination half-life; Tₘₐₓ = time to reach maximum plasma concentration; 90% CI = 90 percent confidence interval; %CV = Percent coefficient of variation; %MR = Geometric least-squares mean ratio. | | | |

### Example 4: Naltrexone and Bupropion Food Effect Study

A study is conducted to assess the plasma pharmacokinetics of NB tablets under fasted and fed conditions. The treatment groups consist of patients who are instructed to: (1) take a single dose of two NB 8/90 tablets without food; (2) take a single dose of two NB 8/90 tablets with food; or (3) take two NB 8/90 tablets with a food during a steady state of NB 8/90 administration. Blood samples are collected for the determination of plasma concentrations for naltrexone, bupropion, and their respective metabolites predose (baseline) and at designated time points postdose.

When NB 8/90 is taken with food, the AUC and Cₘₐₓ for naltrexone increase significantly. When NB 8/90 is taken with food during a steady state of NB 8/90 administration, the AUC and Cₘₐₓ for naltrexone increase significantly.

## Claims

1. A pharmaceutical composition for use in therapy, comprising sustained release formulation of naltrexone or a pharmaceutically acceptable salt thereof and a sustained release formulation of bupropion or a pharmaceutically acceptable salt thereof, wherein said composition is administered with food.

2. The pharmaceutical composition for use according to claim 1, wherein the amount of said bupropion or pharmaceutically acceptable salt thereof is in the range of about 90 mg to about 360 mg per day, and the amount of naltrexone of pharmaceutically acceptable salt thereof is in the range of about 4 mg to about 32 mg per day.

3. The pharmaceutical composition for use according to claim 1, wherein said naltrexone or pharmaceutically acceptable salt thereof comprises a nonsequestered formulation of naltrexone or a pharmaceutically acceptable salt thereof.

4. The pharmaceutical composition for use according to claim 1, wherein said naltrexone or pharmaceutically acceptable salt thereof is administered concurrently with bupropion or a pharmaceutically acceptable salt thereof.

5. The pharmaceutical composition for use according to claim 1, wherein said naltrexone or pharmaceutically acceptable salt thereof is administered prior to or subsequent to bupropion or a pharmaceutically acceptable salt thereof.

6. The pharmaceutical composition for use according to claim 1, wherein said naltrexone or pharmaceutically acceptable salt thereof and said bupropion or pharmaceutically acceptable salt thereof are in a single dosage form.

7. The pharmaceutical composition for use according to any one of the preceding claims, wherein said food comprises a high-fat meal.

8. The pharmaceutical composition for use according to any one of claims 1 to 7, wherein said food comprises a meal selected from the group consisting of a moderate-calorie, moderate-fat meal of about 575 calories and fat accounting for about 23% of the total calorie content; a high-calorie, high-fat meal of about 1000 calories and fat accounting for about 50% of the total calorie content; and a meal that falls within a range defined by said moderate-calorie, moderate fat meal and said high-calorie, high-fat meal.

9. The pharmaceutical composition for use according to any one of claims 1 to 8, wherein said food comprises a moderate-calorie, moderate-fat meal.

10. The pharmaceutical composition for use according to any one of the preceding claims, wherein the treatment schedule for said naltrexone or pharmaceutically acceptable salt thereof and said bupropion or pharmaceutically acceptable salt thereof is:
about 8 mg of said naltrexone or a pharmaceutically acceptable salt thereof and about 90 mg of said bupropion or a pharmaceutically acceptable salt thereof for the first week of treatment;
about 16 mg of said naltrexone or a pharmaceutically acceptable salt thereof and about 180 mg of said bupropion or a pharmaceutically acceptable salt thereof for the second week of treatment;
about 24 mg of said naltrexone or a pharmaceutically acceptable salt thereof and about 270 mg of said bupropion or a pharmaceutically acceptable salt thereof for the third week of treatment; and
about 32 mg of said naltrexone or a pharmaceutically acceptable salt thereof and about 360 mg of said bupropion or a pharmaceutically acceptable salt thereof for the fourth and any subsequent weeks of treatment.

11. The pharmaceutical composition for use according to any one of the preceding claims, wherein said naltrexone or pharmaceutically acceptable salt thereof and said bupropion or pharmaceutically acceptable salt thereof are administered as two 8 mg tablets of sustained-release naltrexone twice daily and two 90 mg tablets of sustained release bupropion twice daily.

12. The pharmaceutical composition for use according to any one of the preceding claims, wherein said naltrexone or pharmaceutically acceptable salt thereof and said bupropion or pharmaceutically acceptable salt thereof are administered for at least 28 weeks.

13. The pharmaceutical composition for use according to any one of the preceding claims, wherein said naltrexone or pharmaceutically acceptable salt thereof and said bupropion or pharmaceutically acceptable salt thereof are administered for at least 56 weeks.

14. The pharmaceutical composition for use according to any one of the preceding claims, wherein the composition is for use in affecting weight loss, reducing cardiovascular risk factors, increasing insulin sensitivity, reducing food cravings, treating visceral fat conditions, mitigating weight gain or promoting weight loss during smoking cessation, and providing weight loss therapy in patients with major depression.

## Patentansprüche

1. Pharmazeutische Zusammensetzung zur Verwendung bei der Therapie, umfassend eine Retardformulierung von Naltrexon oder einem pharmazeutisch annehmbaren Salz davon oder eine Retardformulierung von Bupropion oder einem pharmazeutisch annehmbaren Salz davon, wobei die Zusammensetzung mit Nahrung verabreicht wird.

2. Pharmazeutische Zusammensetzung zur Verwendung nach Anspruch 1, wobei die Menge des Bupropions oder pharmazeutisch annehmbaren Salzes davon im Bereich von etwa 90 mg bis etwa 360 mg pro Tag liegt und die Menge des Naltrexons oder pharmazeutisch annehmbaren Salzes davon im Bereich von etwa 4 mg bis etwa 32 mg pro Tag liegt.

3. Pharmazeutische Zusammensetzung zur Verwendung nach Anspruch 1, wobei das Naltrexon oder pharmazeutisch annehmbare Salz davon eine nicht sequestrierte Formulierung von Naltrexon oder einem pharmazeutisch annehmbaren Salz davon umfasst.

4. Pharmazeutische Zusammensetzung zur Verwendung nach Anspruch 1, wobei das Naltrexon oder pharmazeutisch annehmbare Salz davon gleichzeitig mit Bupropion oder einem pharmazeutisch annehmbaren Salz davon verabreicht wird.

5. Pharmazeutische Zusammensetzung zur Verwendung nach Anspruch 1, wobei das Naltrexon oder pharmazeutisch annehmbare Salz davon vor oder nach Bupropion oder einem pharmazeutisch annehmbaren Salz davon verabreicht wird.

6. Pharmazeutische Zusammensetzung zur Verwendung nach Anspruch 1, wobei das Naltrexon oder pharmazeutisch annehmbare Salz davon und das Bupropion oder pharmazeutisch annehmbare Salz davon in einer Einzeldosisform vorliegen.

7. Pharmazeutische Zusammensetzung zur Verwendung nach einem der vorhergehenden Ansprüche, wobei die Nahrung eine fettreiche Mahlzeit umfasst.

8. Pharmazeutische Zusammensetzung zur Verwendung nach einem der Ansprüche 1 bis 7, wobei die Nahrung eine Mahlzeit umfasst, die aus der Gruppe bestehend aus einer Mahlzeit mit mittlerem Kaloriengehalt und mittlerem Fettgehalt von etwa 575 Kalorien, wobei Fett etwa 23 % des Gesamtkaloriengehalts ausmacht; einer Mahlzeit mit hohem Kaloriengehalt und hohem Fettgehalt von etwa 1000 Kalorien, wobei Fett etwa 50 % des Gesamtkaloriengehalts ausmacht; und einer Mahlzeit, die in einen Bereich fällt, der durch die Mahlzeit mit mittlerem Kaloriengehalt, mittlerem Fettgehalt und die Mahlzeit mit hohem Kaloriengehalt und hohem Fettgehalt definiert ist; ausgewählt ist.

9. Pharmazeutische Zusammensetzung zur Verwendung nach einem der Ansprüche 1 bis 8, wobei die Nahrung eine Mahlzeit mit mittlerem Kaloriengehalt und mittlerem Fettgehalt umfasst.

10. Pharmazeutische Zusammensetzung zur Verwendung nach einem der vorhergehenden Ansprüche, wobei der Behandlungsplan für das Naltrexon oder pharmazeutisch annehmbare Salz davon und das Bupropion oder pharmazeutisch annehmbare Salz davon wie folgt lautet:
etwa 8 mg des Naltrexons oder eines pharmazeutisch annehmbaren Salzes davon und etwa 90 mg des Bupropions oder eines pharmazeutisch unbedenklichen Salzes davon für die erste Behandlungswoche;
etwa 16 mg des Naltrexons oder eines pharmazeutisch annehmbaren Salzes davon und etwa 180 mg des Bupropions oder eines pharmazeutisch unbedenklichen Salzes davon für die zweite Behandlungswoche;
etwa 24 mg des Naltrexons oder eines pharmazeutisch annehmbaren Salzes davon und etwa 270 mg des Bupropions oder eines pharmazeutisch unbedenklichen Salzes davon für die dritte Behandlungswoche; und
etwa 32 mg des Naltrexons oder eines pharmazeutisch annehmbaren Salzes davon und etwa 360 mg des Bupropions oder eines pharmazeutisch unbedenklichen Salzes davon für die vierte Behandlungswoche und alle darauffolgenden Behandlungswochen.

11. Pharmazeutische Zusammensetzung zur Verwendung nach einem der vorhergehenden Ansprüche, wobei das Naltrexon oder pharmazeutisch annehmbare Salz davon und das Bupropion oder pharmazeutisch annehmbare Salz davon als zwei 8-mg-Tabletten von Retard-Naltrexon zweimal täglich und zwei 90-mg-Tabletten Retard-Bupropion zweimal täglich verabreicht werden.

12. Pharmazeutische Zusammensetzung zur Verwendung nach einem der vorhergehenden Ansprüche, wobei das Naltrexon oder pharmazeutisch annehmbare Salz davon und das Bupropion oder pharmazeutisch annehmbare Salz davon für mindestens 28 Wochen verabreicht werden.

13. Pharmazeutische Zusammensetzung zur Verwendung nach einem der vorhergehenden Ansprüche, wobei das Naltrexon oder pharmazeutisch annehmbare Salz davon und das Bupropion oder pharmazeutisch annehmbare Salz davon für mindestens 56 Wochen verabreicht werden.

14. Pharmazeutische Zusammensetzung zur Verwendung nach einem der vorhergehenden Ansprüche, wobei die Zusammensetzung für die Verwendung bei der Beeinflussung von Gewichtsverlust, der Verringerung von kardiovaskulären Risikofaktoren, der Erhöhung der Insulinempfindlichkeit, der Verringerung von Heißhunger, viszeralen Fettleiden, Abschwächung von Gewichtsverlust oder Verhinderung von Gewichtsverlust bei der Raucherentwöhnung und der Bereitstellung von Gewichtsverlusttherapie bei Patienten mit schwerer Depression vorgesehen ist.

## Revendications

1. Composition pharmaceutique pour une utilisation en thérapie, comprenant une formulation à libération prolongée de naltrexone ou d'un sel pharmaceutiquement acceptable correspondant et une formulation à libération prolongée de bupropion ou d'un sel pharmaceutiquement acceptable correspondant, ladite composition étant administrée avec un produit alimentaire.

2. Composition pharmaceutique pour une utilisation selon la revendication 1, la quantité dudit bupropion ou du sel pharmaceutiquement acceptable correspondant étant dans la plage d'environ 90 mg à environ 360 mg par jour, et la quantité de naltrexone ou du sel pharmaceutiquement acceptable correspondant étant dans la plage d'environ 4 mg à environ 32 mg par jour.

3. Composition pharmaceutique pour une utilisation selon la revendication 1, ledit naltrexone ou sel pharmaceutiquement acceptable correspondant comprenant une formulation non séquestrée de naltrexone ou d'un sel pharmaceutiquement acceptable correspondant.

4. Composition pharmaceutique pour une utilisation selon la revendication 1, ledit naltrexone ou sel pharmaceutiquement acceptable correspondant étant administré concurremment avec le bupropion ou un sel pharmaceutique acceptable correspondant.

5. Composition pharmaceutique pour une utilisation selon la revendication 1, ledit naltrexone ou sel pharmaceutiquement acceptable correspondant étant administré avant ou après le bupropion ou un sel pharmaceutique acceptable correspondant.

6. Composition pharmaceutique pour une utilisation selon la revendication 1, ledit naltrexone ou sel pharmaceutiquement acceptable correspondant et ledit bupropion ou un sel pharmaceutiquement acceptable correspondant étant sous une forme de dosage unique.

7. Composition pharmaceutique pour une utilisation selon l'une quelconque des revendications précédentes, ledit produit alimentaire comprenant une farine à teneur élevée en graisses.

8. Composition pharmaceutique pour une utilisation selon l'une quelconque des revendications 1 à 7, ledit produit alimentaire comprenant une farine choisie dans le groupe constitué par une farine à teneur modérée en calories, à teneur modérée en graisses d'environ 575 calories et la graisse comptant pour environ 23 % de la teneur totale en calories ; une farine à teneur élevée en calories, à teneur élevée en graisses d'environ 1 000 calories et la graisse comptant pour environ 50 % de la teneur totale en calories ; et une farine qui tombe dans une plage définie par ladite farine à teneur modérée en calories, à teneur modérée en graisses et ladite farine à teneur élevée en calories, à teneur élevée en graisses.

9. Composition pharmaceutique pour une utilisation selon l'une quelconque des revendications 1 à 8, ledit produit alimentaire comprenant une farine à teneur modérée en calories, à teneur modérée en graisses.

10. Composition pharmaceutique pour une utilisation selon l'une quelconque des revendications précédentes, le programme de traitement pour ledit naltrexone ou le sel pharmaceutiquement acceptable correspondant et ledit bupropion ou le sel pharmaceutiquement acceptable correspondant étant :
environ 8 mg dudit naltrexone ou d'un sel pharmaceutiquement acceptable correspondant et environ 90 mg dudit bupropion ou d'un sel pharmaceutiquement acceptable correspondant pour la première semaine de traitement ;
environ 16 mg dudit naltrexone ou d'un sel pharmaceutiquement acceptable correspondant et environ 180 mg dudit bupropion ou d'un sel pharmaceutiquement acceptable correspondant pour la deuxième semaine de traitement ;
environ 24 mg dudit naltrexone ou d'un sel pharmaceutiquement acceptable correspondant et environ 270 mg dudit bupropion ou d'un sel pharmaceutiquement acceptable correspondant pour la troisième semaine de traitement ; et
environ 32 mg dudit naltrexone ou d'un sel pharmaceutiquement acceptable correspondant et environ 360 mg dudit bupropion ou d'un sel pharmaceutiquement acceptable correspondant pour la quatrième semaine de traitement et de quelconques semaines subséquentes de traitement.

11. Composition pharmaceutique pour une utilisation selon l'une quelconque des revendications précédentes, ledit naltrexone ou le sel pharmaceutiquement acceptable correspondant et ledit bupropion ou le sel pharmaceutiquement acceptable correspondant étant administrés en tant que deux comprimés de 8 mg de naltrexone à libération prolongée deux fois par jour et deux comprimés de 90 mg à libération prolongée de bupropion deux fois par jour.

12. Composition pharmaceutique pour une utilisation selon l'une quelconque des revendications précédentes, ledit naltrexone ou le sel pharmaceutiquement acceptable correspondant et ledit bupropion ou le sel pharmaceutiquement acceptable correspondant étant administrés pendant au moins 28 semaines.

13. Composition pharmaceutique pour une utilisation selon l'une quelconque des revendications précédentes, ledit naltrexone ou le sel pharmaceutiquement acceptable correspondant et ledit bupropion ou le sel pharmaceutiquement acceptable correspondant étant administrés pendant au moins 56 semaines.

14. Composition pharmaceutique pour une utilisation selon l'une quelconque des revendications précédentes, la composition étant pour une utilisation pour affecter une perte de poids, réduire des facteurs de risque cardiovasculaire, augmenter la sensibilité à l'insuline, réduire des envies alimentaires, traiter des affections de graisse viscérale, atténuer un gain de poids ou favoriser une perte de poids pendant un arrêt de tabagisme et fournir une thérapie de perte de poids chez des patients atteints de dépression majeure.
